(19) Europäisches Patentamt
European Patent Office
Office européen des brevets

(11) EP 4 337 095 B1

(12) **EUROPEAN PATENT SPECIFICATION**

(45) Date of publication and mention of the grant of the patent:
**26.08.2026 Bulletin 2026/35**

(21) Application number: **22751889.1**

(22) Date of filing: **05.07.2022**

(51) International Patent Classification (IPC):
***A61B 5/145*** (2006.01) ***A61B 5/1455*** (2006.01)
***A61B 5/1495*** (2006.01) ***A61B 5/00*** (2006.01)

(52) Cooperative Patent Classification (CPC):
**A61B 5/14546; A61B 5/14553; A61B 5/1495; A61B 5/6814; A61B 5/7203**

(86) International application number:
**PCT/US2022/036086**

(87) International publication number:
**WO 2023/283171 (12.01.2023 Gazette 2023/02)**

(54) **METHOD AND APPARATUS FOR NON-INVASIVELY MEASURING BLOOD CIRCULATORY HEMOGLOBIN ACCOUNTING FOR HEMODYNAMIC CONFOUNDERS**

VERFAHREN UND VORRICHTUNG ZUR NICHTINVASIVEN MESSUNG DES BLUTKREISLAUFHÄMOGLOBINS UNTER BERÜCKSICHTIGUNG HÄMODYNAMISCHER KONUNTERRICHTER

PROCÉDÉ ET APPAREIL DE MESURE NON INVASIVE DE L’HÉMOGLOBINE SANGUINE CIRCULATOIRE EN TENANT COMPTE DES FACTEURS DE CONFUSION HÉMODYNAMIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.07.2021 US 202163218684 P**

(43) Date of publication of application:
**20.03.2024 Bulletin 2024/12**

(73) Proprietor: **Becton, Dickinson and Company**
**Franklin Lakes, NJ 07417-1880 (US)**

(72) Inventors:
- **BENNI, Paul, B.**
  **Irvine, CA 92614 (US)**
- **AGUIRRE, Andres, S.**
  **Irvine, CA 92614 (US)**
- **ALBANESE, Antonio**
  **Irvine, CA 92614 (US)**

(74) Representative: **Venner, Julia Ann et al**
**Kilburn & Strode LLP**
**Lacon London**
**84 Theobalds Road**
**London WC1X 8NL (GB)**

(56) References cited:
**CN-A- 107 613 851** **US-A1- 2011 105 912**
**US-A1- 2020 033 258**

- **DESEBBE OLIVIER ET AL: "Tissue Hemoglobin Monitoring Is Unable to Follow Variations of Arterial Hemoglobin During Transitions From Pulsatile to Constant Flow in Cardiac Surgery", JOURNAL OF CARDIOTHORACIC AND VASCULAR ANESTHESIA, vol. 28, no. 3, 1 June 2014 (2014-06-01), pages 668 - 673, XP028865528, ISSN: 1053-0770, DOI: 10.1053/J.JVCA.2013.06.026**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 337 095 B1

## Description

BACKGROUND

1. Technical Field

This invention relates to methods and apparatus for determining blood circulatory hemoglobin values as defined in the appended claims.

2. Background Information

The molecule that carries the oxygen in the blood is hemoglobin. Oxygenated hemoglobin is called oxyhemoglobin ($HbO_2$) and deoxygenated hemoglobin is deoxyhemoglobin (Hb). In some instances, blood may contain other types of hemoglobin (e.g., carboxyhemoglobin (COBb), methemoglobin (MetHb), etc.), but typically in relatively small amounts. The term "total hemoglobin" (THb) as used herein, therefore, refers to the sum of $HbO_2$ and Hb, and is proportional to relative blood volume changes, provided that the hematocrit or hemoglobin concentration of the blood is unchanged. The mammalian cardiovascular system consists of a blood pumping mechanism (the heart), a blood transportation system (blood vessels), and a blood oxygenation system (the lungs). Blood oxygenated by the lungs passes through the heart and is pumped into the arterial vascular system. Under normal conditions, oxygenated arterial blood consists predominately of $HbO_2$. Large arterial blood vessels branch off into smaller branches called arterioles, which profuse throughout biological tissue. The arterioles branch off into capillaries, the smallest blood vessels. In the capillaries, oxygen carried by hemoglobin is transported to the cells in the tissue, resulting in the release of oxygen molecules ($HbO2 \rightarrow Hb$). Under normal conditions, only a fraction of the $HbO_2$ molecules give up oxygen to the tissue, depending on the cellular metabolic need. The capillaries then combine together into venuoles, the beginning of the venous circulatory system. Venuoles then combine into larger blood vessels called veins. The veins further combine and return to the heart, and then venous blood is pumped to the lungs. In the lungs, deoxygenated hemoglobin Hb collects oxygen becoming $HbO_2$ again and the circulatory process is repeated.

Near-infrared spectroscopy (NIRS) is an optical spectrophotometric method of continually monitoring tissue parameters (e.g., oxygen saturation, hemoglobin levels, etc.) that does not require pulsatile blood volume to calculate parameters of clinical value. NIRS spectroscopy is based on the principle that light in the near-infrared range (700 to 1,000 nm) can pass easily through skin, bone, and other tissues where it encounters hemoglobin located mainly within micro-circulation passages (e.g., capillaries, arterioles, and venuoles). Hemoglobin exposed to light in the near infra-red range has specific absorption spectra that varies depending on its oxidation state (i.e., oxyhemoglobin ($HbO_2$) and deoxyhemoglobin (Hb) each act as a distinct chromophore). By using light sources that transmit near-infrared light at specific different wavelengths, and measuring changes in transmitted or reflected light attenuation, concentration changes of the oxyhemoglobin ($HbO_2$) and deoxyhemoglobin (Hb) within tissue can be monitored, as well as oxygen saturation. United States Patent Nos. 6,456,862; 7,072,701; 8,078,250; and 8,396,526 all describe NIRS spectroscopy devices and methods.

Near Infrared spectroscopy (NIRS) oximeters can provide a non-invasively determined total hemoglobin value (THb) for a subject's tissue. As will be described below, the total hemoglobin of tissue is proportional to relative blood volume within the sensed tissue (which volume may change), provided that the hematocrit or hemoglobin concentration of the blood is unchanged. Using an optical based sensor placed on the skin of a subject, a NIRS tissue oximeter can be used to interrogate tissue with different wavelengths of light (e.g., emit light into and detect light emanating from the tissue), and then process the detected light to calculate a total hemoglobin value for the tissue, and if also desired a tissue oxygen saturation ($StO_2$) value. For example, a sensor portion of a NIRS tissue oximeter placed on the forehead of a subject may be used to spectrophotometrically interrogate a subject's brain tissue and thereafter determine total hemoglobin and $StO_2$ values for the subject's brain tissue.

Historically, circulatory blood hemoglobin values (i.e., a hemoglobin value representative of hemoglobin within circulatory blood) have been determined using an invasively drawn blood sample. The invasively drawn blood sample specimen may be analyzed using a CO-oximeter or a blood-gas analyzer. A CO-oximeter is a device that may be operated to measure one or more types of hemoglobin present within a blood specimen; e.g., $HbO_2$, Hb, carboxyhemoglobin (COHb), methemoglobin (MetHb), etc. Most CO-oximeters are spectrophotometric devices that may be operated to determine the presence and amount of the respective types of hemoglobin (e.g., $HbO_2$, Hb, COBb, MetHb, etc.) within the invasively drawn blood sample by measuring the absorption of light at specific wavelengths passing through the blood sample. The relative amounts of absorption at the different wavelengths enable a measurement of the respective types of hemoglobin present within the blood sample. Most blood-gas analyzers, in contrast, are electrochemical type analysis devices that use electrodes and changes in electrical current or potential to detect and measure constituents within the invasively drawn blood sample.

A primary difference between known NIRS tissue oximeters and CO-oximeters or blood-gas analyzers is that known NIRS tissue oximeters are configured to determine a parameter value (e.g., hemoglobin, oxygen saturation, etc.) within tissue, whereas the CO-oximeters or blood-gas analyzers are configured to determine the same parameter value within a circulatory blood sample (i.e., an invasively collected blood sample). Using total hemoglobin as an example parameter, the total hemoglobin value determined within tissue using a known NIRS tissue oximeter can be affected by several different hemodynamic parameters, including hemoglobin concentration per volume of tissue, vasoreactivity, cardiac output, blood flow, partial pressure of carbon dioxide in arterial blood (PaCO2), heart rate, blood volume, hematomas, hyperemia, blood pressure, etc. A total hemoglobin value of a circulatory blood sample determined using a CO-oximeter or a blood-gas analyzer will not be affected by these hemodynamic parameters but requires an invasive collection step. The document DESEBBE OLIVIER ET AL: "Tissue Hemoglobin Monitoring Is Unable to Follow Variations of Arterial Hemoglobin During Transitions From Pulsatile to Constant Flow in Cardiac Surgery",JOURNAL OF CARDIOTHORACIC AND VASCULAR ANESTHESIA, vol. 28, no. 3, 1 June 2014 (2014-06-01), pages 668-673 is relevant prior art for the present invention.

SUMMARY

According to an aspect of the present disclosure, a method of non-invasively measuring tissue hemoglobin of a subject according to claim 1 is provided. The method includes: a) non-invasively sensing tissue of a subject using a near infrared spectrophotometric (NIRS) sensing device, and determining at least one NIRS tissue THb value based on the non-invasive sensing; b) determining whether at least one Hb confounding factor is present during the non-invasive tissue sensing with the NIRS sensing device; and c) determining a NIRS circulatory THb portion of the NIRS tissue THb value based on the presence of the at least one Hb confounding factor during the non-invasive tissue sensing with the NIRS sensing device.

In any of the aspects or embodiments described above and herein, the step of determining whether at least one Hb confounding factor is present during the non-invasive tissue sensing with the NIRS sensing device may include using a hemodynamic measuring device to measure a hemodynamic parameter of the subject at about a same period of time as when the NIRS sensing device is used to non-invasively sensing the tissue of the subject.

In any of the aspects or embodiments described above and herein, the hemodynamic parameter may be a heart rate, a cardiac output, a blood pressure, or a level of vasoreactivity of the subject, or a blood carbon dioxide level within the subject.

The step of determining a NIRS circulatory THb portion of the NIRS tissue THb value includes determining a portion of the NIRS tissue THb value attributable to the Hb confounding factor and accounting for the portion of the NIRS tissue THb value attributable to the Hb confounding factor.

In any of the aspects or embodiments described above and herein, the step of non-invasively sensing tissue of the subject using a NIRS sensing device and the step of determining whether said at least one Hb confounding factor is present during the non-invasive tissue sensing may be both performed using the NIRS sensing device.

In any of the aspects or embodiments described above and herein, the step of non-invasively sensing tissue of the subject using a NIRS sensing device may be performed using a NIRS sensing device that is calibrated using at least one blood circulatory THb value.

In any of the aspects or embodiments described above and herein, the step of non-invasively sensing tissue of the subject using a NIRS sensing device may be performed using a NIRS sensing device that is calibrated using empirical data including blood circulatory THb values.

According to an aspect of the present disclosure, a system for non-invasively measuring tissue hemoglobin of a subject according to claim 7 is provided. The system includes a hemodynamic measuring device and a near infrared spectrophotometric (NIRS) sensing device. The hemodynamic measuring device is configured to sense a hemodynamic parameter and produce signal data representative of the hemodynamic parameter. The NIRS sensing device has at least one transducer and a controller. The transducer has at least one light source and at least one light detector. The controller has at least one processor in communication with the at least one transducer and a memory device having stored instructions. The instructions when executed cause the processor to: a) control the NIRS sensing device to non-invasively sense tissue of a subject and determine at least one NIRS tissue THb value based on the non-invasive sensing; b) determine whether at least one Hb confounding factor is present during the non-invasive tissue sensing using signal data produced by the hemodynamic measuring device; and c) determine a NIRS circulatory THb portion of the NIRS tissue THb value, the determination accounting for the presence of the at least one Hb confounding factor during the non-invasive tissue sensing with the NIRS sensing device.

In any of the aspects or embodiments described above and herein, the system may be configured to cause the hemodynamic measuring device to sense the hemodynamic parameter at about a same period of time as when the NIRS sensing device is used to non-invasively sensing the tissue of the subject.

The instructions that cause the processor to determine the NIRS circulatory THb portion of the NIRS tissue THb

value also cause the processor to determine a portion of the NIRS tissue THb value attributable to the Hb confounding factor.

In any of the aspects or embodiments described above and herein, the NIRS sensing device may be calibrated using at least one blood circulatory THb value.

In any of the aspects or embodiments described above and herein, the NIRS sensing device may be calibrated using empirical data including blood circulatory THb values.

According to an aspect of the present disclosure, a system for non-invasively measuring tissue hemoglobin of a subject according to claim 10 is provided that includes a near infrared spectrophotometric (NIRS) sensing device. The NIRS sensing device has at least one transducer and a controller. The transducer has at least one light source and at least one light detector. The controller has at least one processor in communication with the at least one transducer and a memory device having stored instructions. The instructions when executed cause the processor to: a) control the NIRS sensing device to non-invasively sense tissue of a subject and produce NIRS signal data representative of at least one NIRS tissue THb value; b) control the NIRS sensing device to determine a hemodynamic parameter and produce HP signal data representative of the hemodynamic parameter; c) determine whether at least one Hb confounding factor is present during the non-invasive tissue sensing using HP signal data; and d) determine a NIRS circulatory THb value based on the NIRS signal data and the presence of the at least one Hb confounding factor.

The foregoing has outlined several aspects of the present invention in order that the detailed description of the invention that follows may be better understood. Additional features and advantages of the invention will be described hereinafter which form the subject of the claims of the invention.

BRIEF DESCRIPTION OF THE DRAWINGS

FIG. 1 is a diagrammatic representation of a NIRS sensing device embodiment according to the present disclosure.
FIG. 1A is a diagrammatic representation of a system embodiment according to the present disclosure.
FIG. 2 is a diagrammatic representation of a NIRS sensing device transducer applied to a subject's head.
FIG. 3 is a diagrammatic representation of a NIRS sensing device transducer.
FIG. 4 is a scatter plot of data points shown on a chart having a Y-axis representing blood circulatory THb values and an X-axis showing NIRS tissue THb values, and a trend line determined from the data points.
FIG. 5 is a scatter plot of data points shown on a chart having a Y-axis representing blood circulatory THb values and an X-axis showing NIRS tissue THb values. The data includes a plurality data sets from different subjects. A trend line is fit to each data set.
FIG. 6 is a scatter plot showing two data points collected in a multi-point subject calibration methodology shown on a chart having a Y-axis representing Blood Circulatory values and an X-axis showing NIRS tissue THb values, and a trend line determined from the data points.
FIG. 7 is a diagrammatic block diagram that illustrates aspects of the present disclosure.

DETAILED DESCRIPTION

To facilitate the present description, the following terms used herein are defined as follows:

- "THb" is used herein to mean the total hemoglobin content, collectively including the various types of hemoglobin that may be present within a blood sample such as oxyhemoglobin ($HbO_2$), deoxyhemoglobin (Hb), carboxyhemoglobin (COHb), methemoglobin (MetHb), etc. Typically, the amount of oxyhemoglobin (HbO2) and deoxyhemoglobin (Hb) in a blood sample are disproportionately greater than the amounts of other types of hemoglobin present within a blood sample. To facilitate the description herein "THb" may be described herein as the sum of $HbO_2$ and Hb.
- "blood circulatory THb" is used herein to mean the total hemoglobin content within a collected blood sample. Because a "blood circulatory THb" value is determined from a collected blood sample, it is independent of any hemodynamic effects that may be present in a subject's tissue.
- "NIRS tissue THb" is used to mean the total hemoglobin content of blood within a tissue sample sensed using a NIRS sensing device that does not account for hemodynamic effects that may be present within the sensed tissue.
- "NIRS circulatory THb" is used to mean the total hemoglobin content of blood within a tissue sample sensed using a NIRS sensing device or system that does account for hemodynamic effects that may be present within the sensed tissue.

As stated above, a primary difference between hemoglobin analysis performed on tissue by a NIRS sensing device (sometimes referred to as a "NIRS oximeter") that does not account for hemodynamic effects and a hemoglobin

analysis performed on a collected blood sample is that hemodynamic effects within the tissue sample can affect the hemoglobin analysis. A NIRS tissue THb value may be affected by hemodynamic effects attributable to hemodynamic parameters including, but not limited to, heart rate (HR), blood pressure (BP), vasoreactivity, cardiac output (CO), blood flow, partial pressure of carbon dioxide in arterial blood (PaCO2), blood volume, hematomas, hyperemia, etc. These hemodynamic parameters are not present within a collected volume of blood. Embodiments of the present disclosure are directed to a method and apparatus for noninvasively measuring circulatory hemoglobin (i.e., "NIRS circulatory THb") that accounts for hemodynamic effects. The present disclosure may be used to determine NIRS circulatory THb data on a periodic basis and/or a continuous basis.

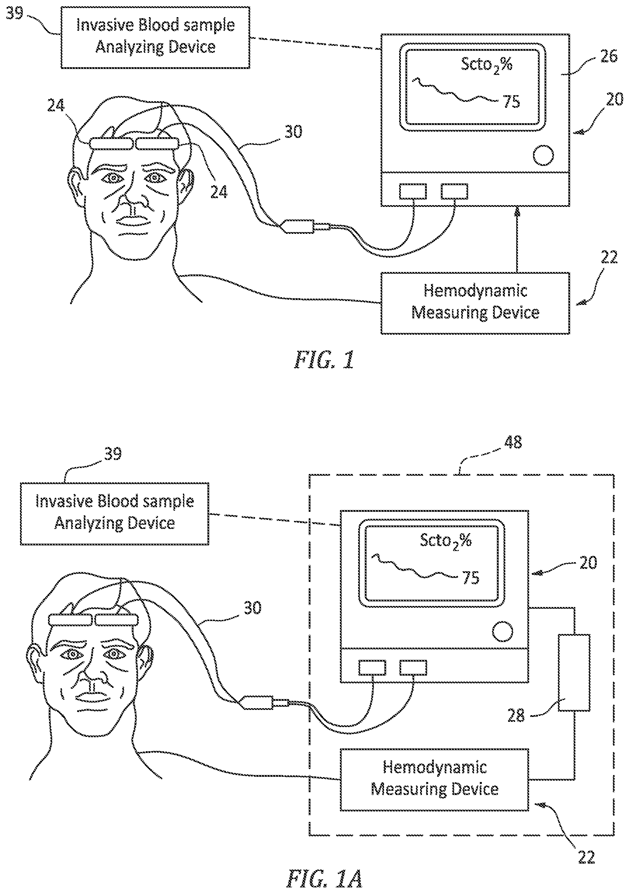

Referring to FIG. 1, aspects of the present disclosure include a NIRS sensing device 20 that is configured to receive signal input from at least one independent hemodynamic measuring device 22, which signal input is representative of a hemodynamic parameter of a subject, and to produce NIRS circulatory THb data therefrom.

Referring to FIG. 1A, other aspects of the present disclosure include a system 48 that includes a NIRS sensing device 20 and at least one hemodynamic measuring device 22 (i.e., a device configured to sense a hemodynamic parameter such as heart rate, blood pressure, vasoreactivity, cardiac output, blood flow, partial pressure of carbon dioxide in arterial blood (PaCO2), etc.) and to produce NIRS circulatory THb data therefrom. The system shown in FIG. 1A includes a system controller 28 in communication with the NIRS sensing device 20 and the hemodynamic measuring device 22.

Other aspects of the present disclosure include a NIRS sensing device 20 that is configured to determine a tissue oxygen parameter (e.g., oxygen saturation values, hemoglobin concentration values, etc.) and at least one hemodynamic parameter, and to produce NIRS circulatory THb data therefrom.

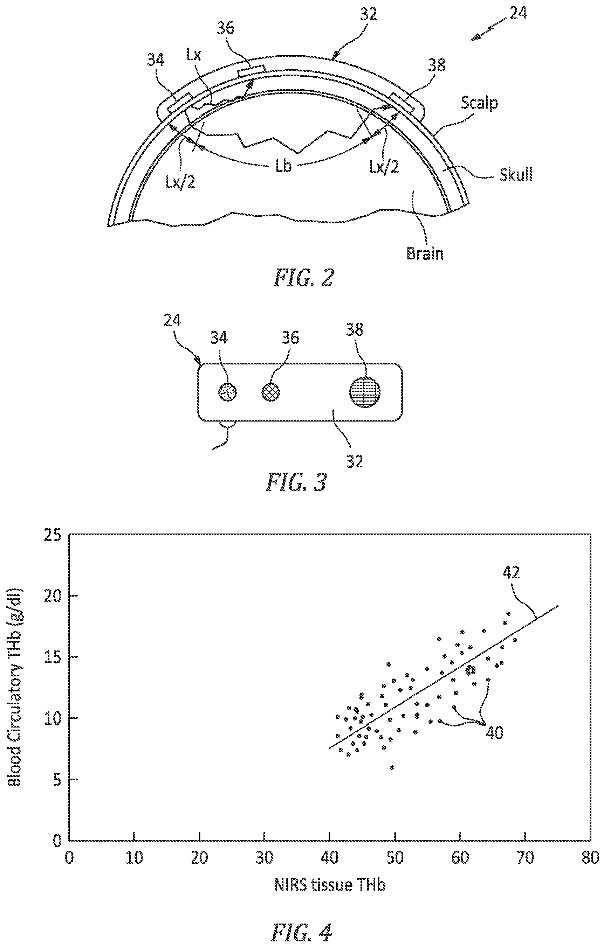

A NIRS sensing device 20 that may be used within the present disclosure has at least one transducer 24 and a controller 26. The transducer 24 may be connected to the controller 26 by a cable 30 (e.g., configured to provide signal communication between the transducer 24 and the controller 26) or the transducer 24 may be in wireless communication with the controller 26. Each transducer 24 includes at least one light source and at least one light detector. FIGS. 2 and 3 illustrate an example of a NIRS sensing device transducer 24 that may be used with the present disclosure. The transducer 24 includes a housing 32, at least one light source 34, and a pair of light detectors 36, 38. The housing 32 is typically configured for attachment directly to a subject's body. The light source 34 and light detectors 36, 38 may be attached to or incorporated within the housing 32. The pair of light detectors 36, 38 may be described as a "near" detector 36 and a "far" detector 38. The terms "near" and "far" indicate the relative distances from the light source 34. The light source 34 may include a plurality of light emitting diodes ("LEDs") that each emit light at a narrow spectral bandwidth at predetermined wavelengths. The light source 34 is not, however, limited to LEDs. The light detectors 36, 38 may each include one or more photodiodes, or other light detecting devices. Non-limiting examples of acceptable NIRS sensing device transducers 24 are described in U.S. Patent Nos. 9,988,873 and 8,428,674. The present disclosure is not limited to any particular transducer configuration.

The NIRS sensing device controller 26 includes one or more processors that can be used to control the operations described in association with any of the computer-implemented method steps described herein. The controller 26 may further include components such as a memory device, an input device, an output device, etc. The term "processor" as used herein may refer to any type of computing device, computational circuit, any type of process or processing circuit, including multiple processors, multicore CPUs, microprocessors, digital signal processors, microcontrollers, or the like, alone or in any combination thereof. The processor(s) included in the controller 26 is capable of executing a series of instructions (e.g., instructions for implementing the method steps / algorithms described herein, controlling components such as the light sources 34 and light detectors 36, 38, etc.) that are stored in memory. The memory is typically a non-transitory memory that may include volatile memory and/or non-volatile memory and may be a computer readable medium. Non-limiting examples of input devices include a keyboard, a pointing device, a touch screen, or the like. Non-limiting examples of an output device include a display unit (e.g., for displaying graphical user interfaces and/or data), or a printer, etc. Features of the present disclosure may be implemented in digital electronic circuitry, in computer hardware, firmware, or any combination thereof. The stored instructions may take the form of a computer program product tangibly embodied in a memory or storage device; e.g., in a machine-readable device accessible for execution by a processor. The controller 26 may be adapted to control operation of the light source 34 and process light signals provided directly or indirectly from the light detectors 36, 38 as described herein. In system embodiments that include a system controller 28, the system controller may be configured as described above.

The controller 26 is adapted to determine blood oxygen parameter values, including oxygen saturation values (that may be referred to as "$SnO_2$", "$StO_2$", "$SctO_2$", "$CrSO_2$", "$rSO_2$", etc.) and hemoglobin concentration values (e.g., $HbO_2$ and Hb) based on the transducer emitted and sensed light. U.S. Patent Nos. 6,456,862; 7,072,701; and 8,396,526 each disclose methods for spectrophotometric blood monitoring. The methods disclosed in U.S. Patent Nos. 6,456,862 and 7,072,701 represent acceptable examples of determining one or more subject-independent blood parameter values. Aspects of the present disclosure may include but are not limited to including those specific methods. The method disclosed in U.S. Patent No. 8,396,526 represents an acceptable example of a method of determining a blood parameter value that accounts for the specific physical characteristics of the particular subject's tissue being sensed; i.e., a subject-

dependent method.

In some embodiments, a NIRS sensing device 20 used within the present disclosure may be calibrated to enable the NIRS sensing device 20 to non-invasively produce NIRS circulatory THb data with greater accuracy; i.e., calibration parameters for the NIRS sensing device 20 may be determined based on empirical data or based on data collected from the particular subject being sensed with the NIRS sensing device 20. Examples of how calibration parameters may be determined based on empirical data or based on data collected from the particular subject are described in U.S. Patent Publication No. 2020/033258. The calibration processes described in U.S. Patent Publication No. 2020/033258 utilize blood circulatory THb data and NIRS tissue THb data. An invasive blood sample analyzing device 39 such as a CO-oximeter or a blood-gas analyzer, or the like may be used to determine a blood circulatory THb value from a collected blood sample. CO-oximeters and blood-gas analyzers are well-known, and no further description is required herein for enablement purposes. Methods and apparatus for collecting NIRS tissue THb data are also known. Non limited examples of methods and apparatus for collecting NIRS tissue THb data are described in U.S. Patent Nos. 6,456,862; 7,072,701; and 8,396,526.

In these calibrated embodiments, the controller 26 may include stored instructions that include one or more calibration parameters representative of empirical data collected from a clinically sufficient population of subjects, or calibration parameters that are individual specific may be determined.

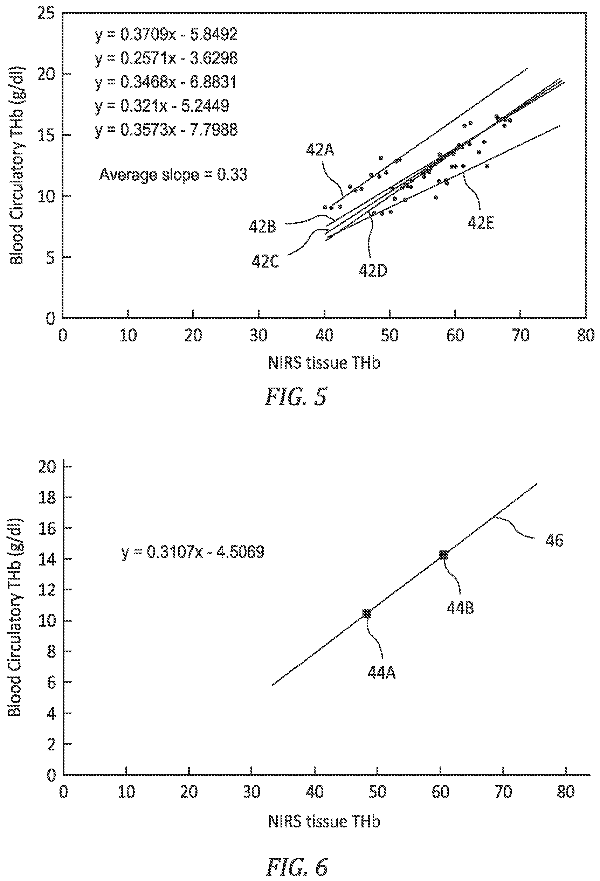

In those embodiments wherein the calibration parameters representative of empirical data collected from a clinically sufficient population of subjects, the empirical data may include a clinically significant number of data sets, with each data set including a NIRS tissue THb value and a corresponding blood circulatory THb value from a subject. Each data set can be plotted as a single data point 40 on a scatter plot (e.g., a chart having a Y-axis representing blood circulatory THb values and an X-axis showing NIRS tissue THb values; See FIG. 4) and a trend line 42 (sometimes referred to as a "best-fit" line) can be determined from the data points 40, which trend line 42 has a slope value and an intercept value. A linear regression technique may be used to define the trend line 42, slope value, and intercept value. The slope value may be referred to as an Empirical Circulatory THb Calibration Slope; e.g., a calibration parameter. In some instances, the empirical data may include a plurality of data sets collected from a subject while the subject is subjected to a stepwise hemodilution protocol. At each step within the hemodilution protocol, a NIRS tissue THb value and a blood circulatory THb value are determined. Each data set (i.e., the NIRS tissue THb value and blood circulatory THb value pair) from a subject subjected to the hemodilution protocol may be plotted as a single point on a scatter plot (e.g., See FIG. 5). A trend line 42A-42E can be fit to the data points from each hemodilution data set (e.g., by linear regression technique), and trend line slope value and an intercept value can be determined for each data set. A clinically acceptable number of slopes can subsequently be used to create a statistically representative slope value (e.g., a mean value) that can be used as an "Empirical Circulatory THb Calibration Slope" value.

Regardless of how the Empirical Circulatory THb Calibration Slope is determined, it may be stored within a non-transitory memory device in communication with the controller 26 of the NIRS sensing device 20 for use in the determination of NIRS circulatory THb data. For example, NIRS circulatory THB data may be determined for a subject using a blood circulatory THb value determined from the subject and a NIRS oximeter having a stored Empirical Circulatory THb Calibration Slope as disclosed in U.S. Patent Publication No. 2020/033258. More specifically, the blood circulatory THb value may be determined from a drawn blood sample analyzed using a CO-oximeter. That blood circulatory THb value may then be input into NIRS sensing device 20 having a stored Empirical Circulatory THb Calibration Slope to determine a "Subject Calibration Intercept". Once the Subject Calibration Intercept is determined, the subject may be sensed to determine a NIRS tissue THb value. The subject's NIRS circulatory THb may then be determined using the NIRS tissue THb value, the Empirical Circulatory THb Calibration Slope, and the Subject Calibration Intercept; e.g., using the following Equation 1:

$$\text{NIRS Circulatory THb} = (\text{NIRS tissue THb} \times \text{Empirical Circulatory THb Calibration Slope}) + \text{Subject Calibration Intercept} \quad \text{(Eqn. 1)}$$

It should be noted that Equation 1 is a non-limiting example of a mathematical expression that can be used. In this way, a NIRS sensing device 20 calibrated in a "subject-independent" manner can be used to determine a NIRS circulatory THb value for a subject.

In those embodiments wherein a NIRS sensing device 20 is calibrated to enable the NIRS sensing device 20 to non-invasively produce NIRS circulatory THb data in a subject-specific manner, the controller 26 may be configured with stored algorithmic instructions to permit a user to enter a plurality of blood circulatory THb values (e.g., from invasively collected blood samples) each associated with a respective NIRS tissue data value (e.g., NIRS tissue THb) both from a particular subject. Each blood circulatory THb value may be determined from a blood sample collected at or about the same time the subject is sensed to produce the respective NIRS tissue data value. Referring to FIG. 6, the blood circulatory THb value and the NIRS tissue THb value from each respective point in time may be represented as a data point 44A, 44B

(i.e., data point 44A represents a blood circulatory THb value and a NIRS tissue THb value from a first point in time, and data point 44B represents a blood circulatory THb value and a NIRS tissue THb value from a second point in time, etc.). FIG. 6 illustrates such data points 44A, 44B plotted on a chart having a Y-axis representing blood circulatory THb values and an X-axis showing NIRS tissue THb values. These data points may be connected via a trend line 46, and a slope and intercept value may be determined. The slope and intercept values may respectively be referred to as an "Individual Subject Calibration Slope" and a "Multi-point Subject Calibration Intercept". If more than two such data points are plotted, the trend line 46 may be determined using a linear regression technique, or a simple slope equation, or the like.

A NIRS sensing device 20 now "calibrated" (via stored algorithm instructions) with the "Individual Subject Calibration Slope" and the "Multi-point Subject Calibration Intercept", may be used to determine a NIRS tissue THb in its normal manner (except that the NIRS sensing device 20 is calibrated). A NIRS Circulatory THb can then be determined, for example, with the following Equation 2:

NIRS Circulatory THb = (NIRS tissue THb × Individual Subject Calibration Slope) + Multi-point Subject Calibration Intercept (Eqn. 2)

It should be noted that Equation 2 is a non-limiting example of a mathematical expression that can be used. The NIRS Circulatory THb could then be determined at any time during monitoring of that particular subject using the calibrated NIRS sensing device 20 without the need for further invasive circulatory blood samples. The present disclosure does not require a NIRS sensing device calibrated in the manner described above.

Embodiments of the present disclosure NIRS sensing device controller 26 (or system controller 28) may be configured to identify the presence or absence of one or more "Hb confounding factors" that may detrimentally affect the accuracy of a determination of a subject's NIRS circulatory THb concentration. The term "Hb confounding factor" as used herein refers to vasoreactivity and/or hemodynamic effects that may confound a NIRS circulatory THb determination. In those embodiments where the NIRS sensing device controller 26 (or system controller 28) is configured to identify the presence or absence of one or more Hb confounding factors, the controller 26, 28 may be configured to account for the Hb confounding factor and thereby mitigate any effect it may have on a NIRS circulatory THb concentration determination.

In some embodiments, the controller 26, 28 may be configured to identify an Hb confounding factor based on empirical data stored within the controller 26, 28. For example, empirical data may be collected that includes NIRS tissue THb data coupled with hemodynamic parameter data such as hemoglobin concentration per volume of tissue, vasoreactivity, cardiac output, blood flow, partial pressure of carbon dioxide in arterial blood (PaCO2), heart rate, blood volume, blood pressure, hematomas, hyperemia, etc. As indicated herein, in some embodiments of the present disclosure the hemodynamic parameter may be measured by a hemodynamic measuring device 22 that is independent of the NIRS sensing device 20. In some of these embodiments, the hemodynamic measuring device 22 and the NIRS sensing device 20 may both be part of a present disclosure system 48 and functionally independent of one another. Within the system 48, the two devices 20, 22 may be in communication with one another or in communication with a common system controller 28. Alternatively, in some of these embodiments the NIRS sensing device 20 may be configured to be in communication with an independent hemodynamic measuring device 22; i.e., the NIRS sensing device 20 may be configured to receive signal data from an independent hemodynamic measuring device 22. In still further embodiments, the NIRS sensing device 20 may be configured to determine both a tissue oxygen parameter (e.g., oxygen saturation values, hemoglobin concentration values, etc.) and at least one hemodynamic parameter (e.g., HR), and to produce NIRS circulatory THb data therefrom. Hence, aspects of the present disclosure permit a determination of NIRS circulatory THb as a function of NIRS tissue THb (calibrated or uncalibrated) and an Hb confounding factor that is associated with hemodynamic parameters such as hemoglobin concentration per volume of tissue, vasoreactivity, cardiac output, blood flow, partial pressure of carbon dioxide in arterial blood (PaCO2), heart rate, blood volume, blood pressure, hematomas, hyperemia, and the like.

$$NIRS\ circulatory\ THb = f(NIRS\ tissue\ THb(cal.\ or\ uncal), HR, BP, CO, PaCO2, etc.) \quad \text{(Eqn. 3)}$$

Using heart rate as an example of a confounding factor, aspects of the present disclosure may utilize an independent hemodynamic measuring device 22 in the form of a HR monitor such as an electrocardiogram ("ECG") or the like, and the controller 26, 28 may include stored empirical data relating to HR levels. The aforesaid empirical data may be developed by sensing a clinically useful number of subjects with a NIRS sensing device 20, a device 39 capable of determining a blood circulatory THb (e.g., a CO-oximeter), and an independent hemodynamic measuring device 22 (e.g., a HR monitor such as an electrocardiogram - "ECG"). Data from these devices can be collected for a predetermined range of heart rates; e.g., heart rates ranging from an "at rest" heart rate to a predetermined elevated heart rate. The collected empirical data may then be analyzed to ascertain what if any hemodynamic effect is associated with the increased heart

rate; e.g., the blood circulatory THb data can be compared to the NIRS tissue THb data at respective heart rates to determine the aforesaid hemodynamic effect. The stored and analyzed empirical data may identify a first portion of NIRS tissue THb data that is attributable to the confounding factor (in this case HR) and a second portion of the NIRS tissue THb data that is attributable to blood circulatory THb.

Regarding blood pressure as an example of a confounding factor, aspects of the present disclosure may utilize an independent blood pressure measuring device 22 (e.g., a ClearSight® system from Edwards Lifesciences Corporation) or the like and the controller 26, 28 may include stored empirical data relating to blood pressure levels. The aforesaid empirical data may be developed by sensing a clinically useful number of subjects with a NIRS sensing device 20, a device 39 capable of determining a blood circulatory THb (e.g., a CO-oximeter), and an independent hemodynamic measuring device 22 (e.g., a blood pressure monitor). Data from these devices can be collected for a predetermined range of blood pressure levels; e.g., blood pressure levels associated with hypertension, hypotension, and normal blood pressure. The collected empirical data may then be analyzed to ascertain what if any hemodynamic effect is associated with blood pressure level; e.g., the blood circulatory THb data can be compared to the NIRS tissue THb data at respective blood pressure levels to determine the aforesaid hemodynamic effect. The stored and analyzed empirical data may identify a first portion of NIRS tissue THb data that is attributable to the confounding factor (e.g., hypertension or hypotension) and a second portion of the NIRS tissue THb data that is attributable to blood circulatory THb.

Regarding cardiac output as an example of a confounding factor, aspects of the present disclosure may utilize an independent hemodynamic measuring device 22 in the form of a cardiac output monitor utilizing doppler echocardiography, or the like, and the controller 26, 28 may include stored empirical data relating to cardiac output levels. The aforesaid empirical data may be developed by sensing a clinically useful number of subjects with a NIRS sensing device 20, a device 39 capable of determining a blood circulatory THb (e.g., a CO-oximeter), and an independent hemodynamic measuring device 22 (e.g., a cardiac output monitor). Data from these devices can be collected for a predetermined range of cardiac outputs; e.g., cardiac outputs ranging from an "at rest" cardiac output to a predetermined elevated cardiac output. The collected empirical data may then be analyzed to ascertain what if any hemodynamic effect is associated with the elevated cardiac output; e.g., the blood circulatory THb data can be compared to the NIRS tissue THb data at respective cardiac output levels to determine the aforesaid hemodynamic effect. The stored and analyzed empirical data may identify a first portion of NIRS tissue THb data that is attributable to the confounding factor (in this case cardiac output) and a second portion of the NIRS tissue THb data that is attributable to blood circulatory THb.

In terms of vasoreactivity as a potential Hb confounding factor, aspects of the present disclosure may utilize an independent hemodynamic measuring device 22 operable to determine vasoreactivity, and the controller 26, 28 may include stored empirical data relating to vasoreactivity. The specific type of hemodynamic measuring device 22 used to determine vasoreactivity will likely depend on the vasoactive stimulus utilized. Devices and methods for determining vasoreactivity are known in the art. The empirical data may be developed by subjecting a clinically useful number of subjects to a regimen of vasoactive stimulus (vasodilatory or vasoconstrictive) while sensing the subjects with a NIRS sensing device 20 to determine NIRS tissue THb data and acquiring blood samples, which blood samples can be analyzed to determine blood circulatory THb data. The NIRS tissue THb data and the blood circulatory THb data can be evaluated at a plurality of different vasoactive stimulus levels (vasodilatory or vasoconstrictive). The collected data may then be analyzed to ascertain what if any hemodynamic effect is attributable to the vasoreactivity; e.g., the blood circulatory THb data can be compared to the NIRS tissue THb data at respective vasoactive levels to determine the hemodynamic effect. The stored and analyzed empirical data may identify a first portion of NIRS tissue THb data is attributable to the confounding factor (in this case vasoreactivity) and a second portion of the NIRS tissue THb data that is attributable to blood circulatory THb.

Carbon dioxide ($CO_2$) within blood affects cerebrovascular reactivity independently of cerebral perfusion pressure. To determine if $CO_2$ may be a confounding factor, aspects of the present disclosure may utilize an independent hemodynamic measuring device 22 in the form of a transcutaneous blood gas monitor (PtcCO2), or an exhaled breath $CO_2$ sensor (EtCO2), or the like, and the controller 26, 28 may include stored empirical data relating to blood $CO_2$ levels. The empirical data may be developed by creating different blood $CO_2$ levels in a clinically useful number of subjects while sensing those subjects with a NIRS sensing device 20 to determine NIRS tissue THb data and acquiring blood samples (analyzed to determine blood circulatory THb data) and sensing the subjects with a blood $CO_2$ measuring device. The NIRS tissue THb data and the blood circulatory THb data can be evaluated at a plurality of different blood $CO_2$ blood levels. The collected data may then be analyzed to ascertain what if any hemodynamic effect is associated with the different blood $CO_2$ levels; e.g., the blood circulatory THb data can be compared to the NIRS tissue THb data at respective blood $CO_2$ levels to determine the hemodynamic effect. The stored and analyzed empirical data may identify a first portion of NIRS tissue THb data is attributable to the confounding factor (in this case $CO_2$) and a second portion of the NIRS tissue THb data that is attributable to blood circulatory THb.

If the NIRS sensing device 20 is operable to provide a pulsatile waveform (e.g., a waveform that reflects pulsatile blood flow), then signals/features may be extracted from the pulsatile waveform that can be used to identify vasoreactivity and/or hemodynamic changes that may confound a NIRS circulatory THb determination. The signals/features that may be

extracted from the pulsatile waveform include: 1) the AC/DC portions of the pulsatile waveform; and 2) the ratio between the energy associated with the cardiac frequency (Ecardiac) and the total energy of the signal (Etotal). These signals contain information relating to the amount of arterial blood that is contained within the tissue volume being sensed by the NIRS sensing device 20. In some embodiments of the present disclosure, empirical data may be developed based on pulsatile waveform signals/features from a clinically useful number of subjects while sensing those subjects with a NIRS sensing device 20 to determine NIRS tissue THb data and acquiring blood samples, which blood samples can be analyzed to determine blood circulatory THb data. The NIRS tissue THb data and the blood circulatory THb data can be evaluated relative to a plurality of different pulsatile waveforms. The collected data may then be analyzed to ascertain what if any hemodynamic effect is attributable to the respective pulsatile waveforms; e.g., the blood circulatory THb data can be compared to the NIRS tissue THb data at respective pulsatile waveforms to determine the hemodynamic effect.

The Hb confounding factor examples provided above are intended to illustrate aspects of the present disclosure to facilitate an appreciation and understanding of the present disclosure, but do not reflect all possible types of Hb confounding factors.

As indicated above, in some embodiments the NIRS sensing device 20 may be configured to determine both a tissue oxygen parameter (e.g., oxygen saturation values, hemoglobin concentration values, etc.) and at least one hemodynamic parameter (e.g., HR), and the NIRS sensing device 20 may include stored empirical data relating to the at least one hemodynamic parameter. Hence, in these embodiments the NIRS sensing device 20 itself may be configured to produce NIRS circulatory THb data (in a manner that accounts for hemodynamic effects), and no independent hemodynamic measuring device is required. A NIRS sensing device 20 that is operable to provide a HR data based on a pulsatile waveform sensed by the NIRS sensing device 20 is a non-limiting example of a NIRS sensing device 20 configured to determine both a tissue oxygen parameter and at least one hemodynamic parameter (e.g., HR). The aforesaid empirical data may be developed by sensing a clinically useful number of subjects with the NIRS sensing device 20 and a device 39 capable of determining a blood circulatory THb (e.g., a CO-oximeter). Data from these devices can be collected for a predetermined range of HRs. The collected empirical data may then be analyzed to ascertain what if any hemodynamic effect is associated with the elevated HR; e.g., the blood circulatory THb data can be compared to the NIRS tissue THb data at respective HRs (determined from the NIRS sensing device 20) to determine the aforesaid hemodynamic effect. The stored and analyzed empirical data may identify a first portion of NIRS tissue THb data is attributable to the confounding factor (in this case HR) and a second portion of the NIRS tissue THb data that is attributable to blood circulatory THb.

The empirical data associated with the various Hb confounding factors indicated above can be used with NIRS tissue THb data and blood circulatory THb data to both identify the presence (or absence) of an Hb confounding factor and if present to account for that Hb confounding factor. The process of accounting for the Hb confounding factor may include identifying a first portion of the NIRS tissue THb data is attributable to the Hb confounding factor and a second portion of the NIRS tissue THb data that is attributable to blood circulatory THb. In this manner, the first portion of the NIRS tissue THb data is attributable to the Hb confounding factor can be separated or distinguished from the second portion of the NIRS tissue THb data that is attributable to blood circulatory THb to permit a determination of the blood THb data (e.g., a NIRS circulatory THb data) that is not subject to hemodynamic effects, or that is only subject to hemodynamic effects in a clinically inconsequential way. Importantly, aspects of the present disclosure system include a non-invasive process that uses empirical data to identify an Hb confounding factor and accounts for such an Hb confounding factor, which process can be performed on a periodic basis and/or a continuous, real-time basis. Prior art techniques that rely upon invasive blood sampling and analysis of the collected blood samples cannot provide THb concentration data on a real-time basis and/or a continuous basis.

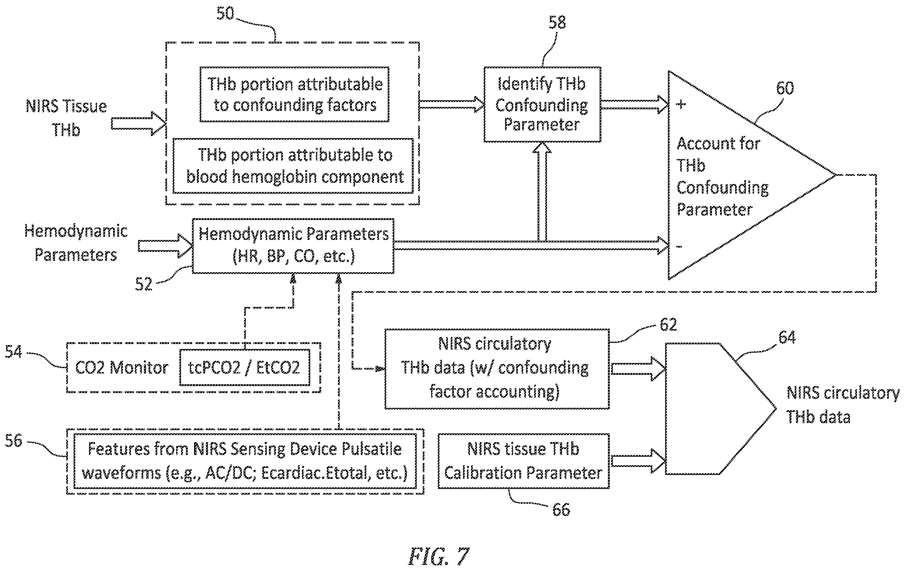

FIG. 7 is a diagrammatic block diagram that illustrates aspects of the present disclosure. NIRS tissue THb is sensed using a NIRS sensing device 20. The signal data (represented as block 50) produced by the NIRS sensing device 20 includes a THb blood hemoglobin component (referred to above as "NIRS tissue THb data attributable to blood circulatory THb") and may include a portion attributable to hemodynamic factors (referred to above as "NIRS tissue THb data attributable to one or more confounding factors") depending on whether hemodynamic factors are present. Block 50 is shown to diagrammatically indicate that these portions may be present within the signal data produced by the NIRS sensing device 20. Below the "NIRS Tissue THb" components, FIG. 7 diagrammatically illustrates a "Hemodynamic Parameters" block 52. This portion of the block diagram represents input from a hemodynamic measuring device 22 configured to sense a hemodynamic parameter. As described above, the hemodynamic measuring device 22 may be a part of a present disclosure system 48 or may be an independent device that is in communication with a present disclosure NIRS sensing device 20 or may be functionally provided within a present disclosure NIRS sensing device 20. The hemodynamic measuring device 22 input and the NIRS sensing device 20 input are utilized to identify the presence or absence of an Hb confounding factor. FIG. 7 also diagrammatically illustrates a $CO_2$ monitor block 54 (another type of hemodynamic measuring device 22) separately from the "Hemodynamic Parameters" block to facilitate the block diagram, and also to make the point that a $CO_2$ monitor may be utilized in some embodiments of the present disclosure but is not required in all embodiments. FIG. 7 also diagrammatically illustrates a "Features from NIRS Sensing Device Pulsatile

Waveforms" block 56 (another type of hemodynamic measuring device 22) separately from the "Hemodynamic Parameters" block to facilitate the block diagram, and also to make the point that such pulsatile waveform features may be utilized in some embodiments of the present disclosure but is not required in all embodiments. FIG. 7 shows a "Identify THb Confounding Parameter" block 58. As described above, embodiments of the NIRS sensing device 20 (or system 48) may be configured to identify the presence (or absence) of a confounding factor based on empirical data. In instances wherein a THb confounding parameter is determined to be present in a form/magnitude that would have a detrimental effect on a NIRS circulatory THb determination, then the present disclosure NIRS sensing device 20 (or system 48) and method may be configured to account for that confounding factor as is shown in the "Account for THb Confounding Parameter" block 60. As indicated above, in some embodiments the "accounting" may include separating or distinguishing the portion of the NIRS tissue THb data attributable to the THb confounding factor from the portion of the NIRS tissue THb data that is attributable to blood circulatory THb. The accounting permits a non-invasive determination of the blood THb data (e.g., a NIRS circulatory THb data) that is either not subject to hemodynamic effects or is only subject to hemodynamic effects in a clinically inconsequential way. An accounting that includes separating or distinguishing the portion of the NIRS tissue THb data attributable to the THb confounding factor is a non-limiting example of accounting for hemodynamic effects. The NIRS circulatory THb data that results from the accounting (shown in block 62 "NIRS circulatory THb data (w/confounding factor accounting") may then be reported (block 64) as the NIRS circulatory THb data. In some embodiments as described above, the NIRS circulatory THb data that results from the accounting may be processed using a calibrated NIRS sensing device 20 as described herein (e.g., see FIGS. 4 and 5 and the description associated therewith) as shown in block 66, and subsequently reported in block 64. In some embodiments, the NIRS tissue data input as shown in block 50 may be produced using a calibrated NIRS sensing device 20 (as described herein - see FIGS. 4 and 5 and the description associated therewith). In these embodiments, processing associate with block 66 may be eliminated.

In some embodiments of the present disclosure, artificial intelligence or more specifically machine learning may be used to facilitate application of the algorithms described herein; e.g., algorithms that account for hemodynamic effects. To illustrate and as described herein, aspects of the present disclosure include an accounting that includes separating or distinguishing the portion of the NIRS tissue Hb data attributable to the Hb confounding factor and the HB confounding factor may be based on empirical data. Machine learning may be used to facilitate that process or others described herein. Machine learning techniques are known, and the present disclosure is not limited to any particular machine learning technique or process.

While the principles of the disclosure have been described above in connection with specific apparatuses and methods, it is to be clearly understood that this description is made only by way of example and not as limitation on the scope of the disclosure. Specific details are given in the above description to provide a thorough understanding of the embodiments. However, it is understood that the embodiments may be practiced without these specific details.

It is noted that the embodiments may be described as a process which is depicted as a flowchart, a flow diagram, a block diagram, etc. Although any one of these structures may describe the operations as a sequential process, many of the operations can be performed in parallel or concurrently. In addition, the order of the operations may be rearranged. A process may correspond to a method, a function, a procedure, a subroutine, a subprogram, etc.

The singular forms "a," "an," and "the" refer to one or more than one, unless the context clearly dictates otherwise. For example, the term "comprising a specimen" includes single or plural specimens and is considered equivalent to the phrase "comprising at least one specimen." The term "or" refers to a single element of stated alternative elements or a combination of two or more elements unless the context clearly indicates otherwise. As used herein, "comprises" means "includes." Thus, "comprising A or B," means "including A or B, or A and B," without excluding additional elements.

It is noted that various connections are set forth between elements in the present description and drawings (the contents of which are included in this disclosure by way of reference). It is noted that these connections are general and, unless specified otherwise, may be direct or indirect and that this specification is not intended to be limiting in this respect. Any reference to attached, fixed, connected or the like may include permanent, removable, temporary, partial, full and/or any other possible attachment option.

The scope of the present invention is limited by the scope of the appended claims.

## Claims

**1.** A method of non-invasively measuring tissue hemoglobin of a subject, comprising:

non-invasively sensing tissue of a subject using a near infrared spectrophotometric (NIRS) sensing device (20), and determining at least one NIRS tissue total hemoglobin (THb) value based on the non-invasive sensing;

determining whether at least one Hb confounding factor is present during the non-invasive tissue sensing with the NIRS sensing device (20), wherein the at least one Hb confounding factor is a vasoreactivity and/or a hemodynamic effect that may confound a NIRS circulatory THb determination; and

determining a NIRS circulatory THb portion of the NIRS tissue THb value based on the presence of the at least one Hb confounding factor during the non-invasive tissue sensing with the NIRS sensing device (20);
wherein THb refers to the sum of $HbO_2$ and Hb; and
wherein the step of determining a NIRS circulatory THb portion of the NIRS tissue THb value includes determining a portion of the NIRS tissue THb value attributable to the Hb confounding factor and accounting for the portion of the NIRS tissue THb value attributable to the Hb confounding factor.

**2.** The method of claim 1, wherein the step of determining whether at least one Hb confounding factor is present during the non-invasive tissue sensing with the NIRS sensing device (20) includes using a hemodynamic measuring device (22) to measure a hemodynamic parameter of the subject at about a same period of time as when the NIRS sensing device (20) is used to non-invasively sensing the tissue of the subject.

**3.** The method of claim 1 or claim 2, wherein the hemodynamic parameter is:

a) a heart rate of the subject;
b) a cardiac output of the subject;
c) a level of vasoreactivity of the subject;
d) a blood carbon dioxide level within the subject; or
e) a blood pressure level of the subject.

**4.** The method of any preceding claim, wherein the step of non-invasively sensing tissue of the subject using a NIRS sensing device (20) and the step of determining whether said at least one Hb confounding factor is present during the non-invasive tissue sensing are both performed using the NIRS sensing device (20).

**5.** The method of any preceding claim, wherein the step of non-invasively sensing tissue of the subject using a NIRS sensing device (20) is performed using a NIRS sensing device (20) that is calibrated using at least one blood circulatory THb value.

**6.** The method of any preceding claim, wherein the step of non-invasively sensing tissue of the subject using a NIRS sensing device (20) is performed using a NIRS sensing device (20) that is calibrated using empirical data including blood circulatory THb values.

**7.** A system (48) for non-invasively measuring tissue hemoglobin of a subject, comprising:

a hemodynamic measuring device (22) configured to sense a hemodynamic parameter and produce signal data representative of the hemodynamic parameter; and
a near infrared spectrophotometric (NIRS) sensing device (20), having at least one transducer (24) and a controller (26), the transducer (24) having at least one light source (34) and at least one light detector (36, 38), the controller (26) having at least one processor in communication with the at least one transducer (24) and a memory device having stored instructions, which instructions when executed cause the processor to:

control the NIRS sensing device (20) to non-invasively sense tissue of a subject and determine at least one NIRS tissue total hemoglobin (THb) value based on the non-invasive sensing;
determine whether at least one Hb confounding factor is present during the non-invasive tissue sensing using signal data produced by the hemodynamic measuring device (22), wherein the at least one Hb confounding factor is a vasoreactivity and/or a hemodynamic effect that may confound a NIRS circulatory THb determination; and
determine a NIRS circulatory THb portion of the NIRS tissue THb value, the determination accounting for the presence of the at least one Hb confounding factor during the non-invasive tissue sensing with the NIRS sensing device (20);
wherein THb refers to the sum of $HbO_2$ and Hb; and
wherein the instructions that cause the processor to determine the NIRS circulatory THb portion of the NIRS tissue THb value also cause the processor to determine a portion of the NIRS tissue THb value attributable to the Hb confounding factor.

**8.** The system of claim 7, wherein the system (48) is configured to cause the hemodynamic measuring device (22) to sense the hemodynamic parameter at about a same period of time as when the NIRS sensing device is used to non-invasively sensing the tissue of the subject.

9. The system of claim 7 or 8, wherein the hemodynamic measuring device (22) is configured to sense:

a) a heart rate of the subject as a hemodynamic parameter;
b) a cardiac output of the subject as a hemodynamic parameter;
c) a level of vasoreactivity of the subject as a hemodynamic parameter;
d) a blood carbon dioxide level of the subject as a hemodynamic parameter;
e) a blood pressure level of the subject as a hemodynamic parameter;

10. A system (48) for non-invasively measuring tissue hemoglobin of a subject, comprising:
a near infrared spectrophotometric (NIRS) sensing device (20), having at least one transducer (24) and a controller (26), the transducer (24) having at least one light source (34) and at least one light detector (36, 38), the controller (26) having at least one processor in communication with the at least one transducer (24) and a memory device having stored instructions, which instructions when executed cause the processor to:

control the NIRS sensing device (20) to non-invasively sense tissue of a subject and produce NIRS signal data representative of at least one NIRS tissue total hemoglobin (THb) value;
control the NIRS sensing device (20) to determine a hemodynamic parameter and produce HP signal data representative of the hemodynamic parameter;
determine whether at least one Hb confounding factor is present during the non-invasive tissue sensing using HP signal data, wherein the at least one Hb confounding factor is a vasoreactivity and/or a hemodynamic effect that may confound a NIRS circulatory THb determination; and
determine a NIRS circulatory THb value based on the NIRS signal data and the presence of the at least one Hb confounding factor;
wherein THb refers to the sum of $HbO_2$ and Hb; and
wherein the instructions that cause the processor to determine the NIRS circulatory THb value based on the NIRS signal data also cause the processor to determine a portion of the NIRS tissue THb value attributable to the Hb confounding factor.

## Patentansprüche

1. Verfahren zum nichtinvasiven Messen von Gewebehämoglobin eines Subjekts, umfassend:

nichtinvasives Erfassen von Gewebe eines Subjekts unter Verwendung einer Nahinfrarot-spektrophotometrischen (NIRS) Erfassungsvorrichtung (20) und Bestimmen wenigstens eines NIRS-Gewebe-Gesamthämoglobin (THb)-Wertes basierend auf der nichtinvasiven Erfassung;
Bestimmen, ob mindestens ein Hb-Störfaktor während der nichtinvasiven Gewebeerfassung mit der NIRS-Erfassungsvorrichtung (20) vorliegt, wobei es sich bei dem mindestens einen Hb-Störfaktor um eine Vasoreaktivität und/oder eine hämodynamische Wirkung handelt, die eine NIRS-Kreislauf-THB-Bestimmung stören kann/können; und
Bestimmen eines NIRS-Kreislauf-THb-Anteils des NIRS-Gewebe-THb-Wertes basierend auf dem Vorliegen des wenigstens einen Hb-Störfaktors während der nichtinvasiven Gewebeerfassung mit der NIRS-Erfassungsvorrichtung (20);
wobei sich THb auf die Summe von $HbO_2$ und Hb bezieht; und wobei der Schritt des Bestimmens eines NIRS-Kreislauf-THb-Anteils des NIRS-Gewebe-THb-Wertes das Bestimmen eines Anteils des NIRS-Gewebe-THb-Wertes, der dem Hb-Störfaktor zuzuschreiben ist, und das Berücksichtigen des Anteils des NIRS-Gewebe-THb-Wertes, der dem Hb-Störfaktor zuzuschreiben ist, beinhaltet.

2. Verfahren nach Anspruch 1, wobei der Schritt des Bestimmens, ob wenigstens ein Hb-Störfaktor während der nichtinvasiven Gewebeerfassung mit der NIRS-Erfassungsvorrichtung (20) vorliegt, beinhaltet, dass eine hämodynamische Messvorrichtung (22) verwendet wird, um einen hämodynamischen Parameter des Subjekts in etwa demselben Zeitraum zu messen, in dem die NIRS-Erfassungsvorrichtung (20) verwendet wird, um das Gewebe des Subjekts nichtinvasiv zu erfassen.

3. Verfahren nach Anspruch 1 oder Anspruch 2, wobei dies der hämodynamische Parameter ist:

a) eine Herzfrequenz des Subjekts;
b) eine Herzleistung des Subjekts;

c) ein Grad der Vasoreaktivität des Subjekts;
d) ein Blutkohlendioxidpegel in dem Subjekt; oder
e) ein Blutdruckniveau des Subjekts.

**4.** Verfahren nach einem der vorstehenden Ansprüche, wobei der Schritt des nichtinvasiven Erfassens von Gewebe des Subjekts unter Verwendung einer NIRS-Erfassungsvorrichtung (20) sowie der Schritt des Bestimmens, ob wenigstens ein Hb-Störfaktor während der nichtinvasiven Gewebeerfassung vorliegt, unter Verwendung der NIRS-Erfassungsvorrichtung (20) durchgeführt werden.

**5.** Verfahren nach einem der vorstehenden Ansprüche, wobei der Schritt des nichtinvasiven Erfassens von Gewebe des Subjekts unter Verwendung einer NIRS-Erfassungsvorrichtung (20) unter Verwendung einer NIRS-Erfassungsvorrichtung (20) durchgeführt wird, die unter Verwendung wenigstens eines Blutkreislauf-THb-Wertes kalibriert ist.

**6.** Verfahren nach einem der vorstehenden Ansprüche, wobei der Schritt des nichtinvasiven Erfassens von Gewebe des Subjekts unter Verwendung einer NIRS-Erfassungsvorrichtung (20) unter Verwendung einer NIRS-Erfassungsvorrichtung (20) durchgeführt wird, die unter Verwendung empirischer Daten kalibriert ist, was Blutkreislauf-THb-Werte einschließt.

**7.** System (48) zum nichtinvasiven Messen von Gewebehämoglobin eines Subjekts, umfassend:

eine hämodynamische Messvorrichtung (22), die dazu ausgelegt ist, einen hämodynamischen Parameter zu erfassen und Signaldaten zu erzeugen, die den hämodynamischen Parameter darstellen; und
eine Nahinfrarot-spektrophotometrische (NIRS) Erfassungsvorrichtung (20), die wenigstens einen Wandler (24) und eine Steuerung (26) aufweist, wobei der Wandler (24) wenigstens eine Lichtquelle (34) und wenigstens einen Lichtdetektor (36, 38) aufweist, wobei die Steuerung (26) wenigstens einen Prozessor aufweist, der in Kommunikationsverbindung mit dem wenigstens einen Wandler (24) steht, und wobei eine Speichervorrichtung gespeicherte Anweisungen aufweist, die, wenn sie ausgeführt werden, den Prozessor hierzu veranlassen:

Steuern der NIRS-Erfassungsvorrichtung (20), um Gewebe eines Subjekts nichtinvasiv zu erfassen und wenigstens einen NIRS-Gewebe-Gesamthämoglobin (THb)-Wert basierend auf der nichtinvasiven Erfassung zu bestimmen;
Bestimmen, ob mindestens ein Hb-Störfaktor während der nichtinvasiven Gewebeerfassung unter Verwendung von durch die hämodynamische Messvorrichtung (22) erzeugten Signaldaten vorliegt, wobei es sich bei dem mindestens einen Hb-Störfaktor um eine Vasoreaktivität und/oder eine hämodynamische Wirkung handelt, die eine NIRS-Kreislauf-THB-Bestimmung stören kann/können; und
Bestimmen eines NIRS-Kreislauf-THb-Anteils des NIRS-Gewebe-THb-Wertes, wobei die Bestimmung das Vorliegen des wenigstens einen Hb-Störfaktors während der nichtinvasiven Gewebeerfassung mit der NIRS-Erfassungsvorrichtung (20) berücksichtigt;
wobei sich THb auf die Summe von $HbO_2$ und Hb bezieht; und wobei die Anweisungen, die den Prozessor veranlassen, den NIRS-Kreislauf-THb-Anteil des NIRS-Gewebe-THb-Wertes zu bestimmen, den Prozessor außerdem veranlassen, einen Anteil des NIRS-Gewebe-THb-Wertes zu bestimmen, der dem Hb-Störfaktor zuzuschreiben ist.

**8.** System nach Anspruch 7, wobei das System (48) dazu ausgelegt ist, die hämodynamische Messvorrichtung (22) zu veranlassen, den hämodynamischen Parameter in etwa demselben Zeitraum zu erfassen, in dem die NIRS-Erfassungsvorrichtung verwendet wird, um das Gewebe des Subjekts nichtinvasiv zu erfassen.

**9.** System nach Anspruch 7 oder 8, wobei die hämodynamische Messvorrichtung (22) dazu ausgelegt ist, dies zu erfassen:

a) eine Herzfrequenz des Subjekts als hämodynamischer Parameter;
b) eine Herzleistung des Subjekts als hämodynamischer Parameter;
c) einen Grad der Vasoreaktivität des Subjekts als hämodynamischer Parameter;
d) einen Blutkohlendioxidpegel des Subjekts als hämodynamischer Parameter;
e) ein Blutdruckniveau des Subjekts als hämodynamischer Parameter;

**10.** System (48) zum nichtinvasiven Messen von Gewebehämoglobin eines Subjekts, umfassend:
eine Nahinfrarot-spektrophotometrische (NIRS) Erfassungsvorrichtung (20), die wenigstens einen Wandler (24) und

eine Steuerung (26) aufweist, wobei der Wandler (24) wenigstens eine Lichtquelle (34) und wenigstens einen Lichtdetektor (36, 38) aufweist, wobei die Steuerung (26) wenigstens einen Prozessor aufweist, der in Kommunikationsverbindung mit dem wenigstens einen Wandler (24) steht, und wobei eine Speichervorrichtung gespeicherte Anweisungen aufweist, die, wenn sie ausgeführt werden, den Prozessor hierzu veranlassen:

Steuern der NIRS-Erfassungsvorrichtung (20), um Gewebe eines Subjekts nichtinvasiv zu erfassen und NIRS-Signaldaten zu erzeugen, die wenigstens einen NIRS-Gewebe-Gesamthämoglobin (THb)-Wert darstellen;
Steuern der NIRS-Erfassungsvorrichtung (20), um einen hämodynamischen Parameter zu bestimmen und HP-Signaldaten zu erzeugen, die den hämodynamischen Parameter darstellen;
Bestimmen, ob mindestens ein Hb-Störfaktor während der nichtinvasiven Gewebeerfassung unter Verwendung von HP-Signaldaten vorliegt, wobei es sich bei dem mindestens einen Hb-Störfaktor um eine Vasoreaktivität und/oder eine hämodynamische Wirkung handelt, die eine NIRS-Kreislauf-THB-Bestimmung stören kann/-können; und
Bestimmen eines NIRS-Kreislauf-THb-Wertes basierend auf den NIRS-Signaldaten und dem Vorliegen des wenigstens einen Hb-Störfaktors;
wobei sich THb auf die Summe von $HbO_2$ und Hb bezieht; und wobei die Anweisungen, die den Prozessor veranlassen, den NIRS-Kreislauf-THb-Wert basierend auf den NIRS-Signaldaten zu bestimmen, den Prozessor außerdem veranlassen, einen Anteil des NIRS-Gewebe-THb-Wertes zu bestimmen, der dem Hb-Störfaktor zuzuschreiben ist.

## Revendications

**1.** Procédé de mesure non invasive de l’hémoglobine tissulaire d’un sujet, comprenant :

la détection non invasive de tissu d’un sujet au moyen d’un dispositif de détection spectrophotométrique proche infrarouge (NIRS) (20), et la détermination d’au moins une valeur d’hémoglobine totale (THb) tissulaire NIRS sur la base de la détection non invasive ;
la détermination si au moins un facteur de confusion Hb est présent pendant la détection tissulaire non invasive avec le dispositif de détection NIRS (20), l’au moins un facteur de confusion Hb étant une vasoréactivité et/ou un effet hémodynamique qui peut perturber une détermination de THb circulatoire NIRS ; et
la détermination d’une partie de THb circulatoire NIRS de la valeur THb tissulaire NIRS sur la base de la présence d’au moins un facteur de confusion Hb pendant la détection tissulaire non invasive avec le dispositif de détection NIRS (20) ;
THb désignant la somme de $HbO_2$ et Hb ; et
l’étape de détermination d’une partie de THb circulatoire NIRS de la valeur de THb tissulaire NIRS comprenant la détermination d’une partie de la valeur de THb tissulaire NIRS attribuable au facteur de confusion Hb et la prise en compte de la partie de la valeur de THb tissulaire NIRS attribuable au facteur de confusion Hb.

**2.** Procédé selon la revendication 1, l’étape de détermination si au moins un facteur de confusion Hb est présent pendant la détection de tissu non invasif avec le dispositif de détection NIRS (20) comprenant l’utilisation d’un dispositif de mesure hémodynamique (22) pour mesurer un paramètre hémodynamique du sujet à environ une même période de temps que lorsque le dispositif de détection NIRS (20) est utilisé pour détecter de manière non invasive le tissu du sujet.

**3.** Procédé selon la revendication 1 ou la revendication 2, le paramètre hémodynamique étant :

a) une fréquence cardiaque du sujet ;
b) un débit cardiaque du sujet ;
c) un niveau de vasoréactivité du sujet ;
d) un niveau de dioxyde de carbone sanguin chez le sujet ; ou
e) un niveau de pression sanguine du sujet.

**4.** Procédé selon l’une quelconque des revendications précédentes, l’étape de détection non invasive de tissu du sujet en utilisant un dispositif de détection NIRS (20) et l’étape de détermination de la présence ou non dudit au moins un facteur de confusion Hb pendant la détection de tissu non invasive étant toutes deux effectuées en utilisant le dispositif de détection NIRS (20).

5. Procédé selon l'une quelconque des revendications précédentes, l'étape de détection non invasive de tissu du sujet en utilisant un dispositif de détection NIRS (20) étant effectuée en utilisant un dispositif de détection NIRS (20) qui est étalonné en utilisant au moins une valeur THb de circulation sanguine.

6. Procédé selon l'une quelconque des revendications précédentes, l'étape de détection non invasive de tissu du sujet en utilisant un dispositif de détection NIRS (20) étant effectuée en utilisant un dispositif de détection NIRS (20) qui est étalonné en utilisant des données empiriques incluant des valeurs THb de la circulation sanguine.

7. Système (48) de mesure non invasive de l'hémoglobine tissulaire d'un sujet, comprenant :

   un dispositif de mesure hémodynamique (22) configuré pour détecter un paramètre hémodynamique et produire des données de signal représentatives du paramètre hémodynamique ; et
   un dispositif de détection spectrophotométrique proche infrarouge (NIRS) (20) comprenant au moins un transducteur (24) et un dispositif de commande (26), le transducteur (24) comprenant au moins une source lumineuse (34) et au moins un détecteur de lumière (36, 38), le dispositif de commande (26) comprenant au moins un processeur en communication avec l'au moins un transducteur (24) et un dispositif de mémoire comprenant des instructions stockées, lesquelles instructions lorsqu'elles sont exécutées, amènent le processeur à :

      commander le dispositif de détection NIRS (20) pour détecter de manière non invasive le tissu d'un sujet et déterminer au moins une valeur d'hémoglobine totale (THb) tissulaire NIRS sur la base de la détection non invasive ;
      déterminer si au moins un facteur de confusion Hb est présent pendant la détection tissulaire non invasive à l'aide de données de signal produites par le dispositif de mesure hémodynamique (22), l'au moins un facteur de confusion Hb étant une vasoréactivité et/ou un effet hémodynamique qui peut perturber une détermination de THb circulatoire NIRS ; et
      déterminer une partie de THb circulatoire NIRS de la valeur de THb tissulaire NIRS, la détermination tenant compte de la présence d'au moins un facteur de confusion Hb pendant la détection tissulaire non invasive avec le dispositif de détection NIRS (20) ;
      THb désignant la somme de $HbO_2$ et Hb ; et
      les instructions qui amènent le processeur à déterminer la partie de THb circulatoire NIRS de la valeur THb tissulaire NIRS amenant également le processeur à déterminer une partie de la valeur THb tissulaire NIRS attribuable au facteur de confusion Hb.

8. Système selon la revendication 7, le système (48) étant configuré pour amener le dispositif de mesure hémodynamique (22) à détecter le paramètre hémodynamique à environ une même période de temps que lorsque le dispositif de détection NIRS est utilisé pour détecter de manière non invasive le tissu du sujet.

9. Système selon la revendication 7 ou 8, le dispositif de mesure hémodynamique (22) étant configuré pour détecter :

   a) une fréquence cardiaque du sujet comme paramètre hémodynamique ;
   b) un débit cardiaque du sujet en tant que paramètre hémodynamique ;
   c) un niveau de vasoréactivité du sujet en tant que paramètre hémodynamique ;
   d) un niveau de dioxyde de carbone sanguin du sujet en tant que paramètre hémodynamique ;
   e) un niveau de pression sanguine du sujet en tant que paramètre hémodynamique ;

10. Système (48) de mesure non invasive de l'hémoglobine tissulaire d'un sujet, comprenant :
un dispositif de détection spectrophotométrique proche infrarouge (NIRS) (20) comprenant au moins un transducteur (24) et un dispositif de commande (26), le transducteur (24) comprenant au moins une source lumineuse (34) et au moins un détecteur de lumière (36, 38), le dispositif de commande (26) comprenant au moins un processeur en communication avec l'au moins un transducteur (24) et un dispositif de mémoire comprenant des instructions stockées, lesquelles instructions lorsqu'elles sont exécutées, amènent le processeur à :

   commander le dispositif de détection NIRS (20) pour détecter de manière non invasive le tissu d'un sujet et produire des données de signal NIRS représentatives d'au moins une valeur d'hémoglobine totale de tissu NIRS (THb) ;
   commander le dispositif de détection NIRS (20) pour déterminer un paramètre hémodynamique et produire des données de signal HP représentatives du paramètre hémodynamique ;
   déterminer si au moins un facteur de confusion Hb est présent pendant la détection tissulaire non invasive à l'aide

des données de signal HP, l'au moins un facteur de confusion Hb étant une vasoréactivité et/ou un effet hémodynamique qui peut perturber une détermination de THb circulatoire NIRS ; et

déterminer une valeur THb circulatoire NIRS en fonction des données du signal NIRS et de la présence d'au moins un facteur de confusion Hb ;

THb désignant la somme de $HbO_2$ et Hb ; et

les instructions qui amènent le processeur à déterminer la valeur THb circulatoire NIRS sur la base des données de signal NIRS amenant également le processeur à déterminer une partie de la valeur THb tissulaire NIRS attribuable au facteur de confusion Hb.

39
Invasive Blood sample Analyzing Device
Scto2%
75
26
20
24
30
24
22
Hemodynamic Measuring Device

*FIG. 1*

48
39
Invasive Blood sample Analyzing Device
Scto2%
75
20
30
28
Hemodynamic Measuring Device
22

*FIG. 1A*

24
32
36
34
Lx
38
Scalp
Skull
Lb
Lx/2
Lx/2
Brain


FIG. 2

24
34
36
38
32


FIG. 3

Blood Circulatory THb (g/dl)
NIRS tissue THb
42
40
0
5
10
15
20
25
0
10
20
30
40
50
60
70
80


FIG. 4

y = 0.3709x - 5.8492
y = 0.2571x - 3.6298
y = 0.3468x - 6.8831
y = 0.321x - 5.2449
y = 0.3573x - 7.7988
Average slope = 0.33
42A
42B
42C
42D
42E
Blood Circulatory THb (g/dl)
NIRS tissue THb
0
5
10
15
20
25
0
10
20
30
40
50
60
70
80


*FIG. 5*

y = 0.3107x - 4.5069
46
44A
44B
Blood Circulatory THb (g/dl)
NIRS tissue THb
0
2
4
6
8
10
12
14
16
18
20
0
10
20
30
40
50
60
70
80


*FIG. 6*

NIRS Tissue THb
50
THb portion attributable to confounding factors
THb portion attributable to blood hemoglobin component
58
Identify THb Confounding Parameter
60
+
Account for THb Confounding Parameter
-
Hemodynamic Parameters
Hemodynamic Parameters (HR, BP, CO, etc.)
52
54
CO2 Monitor
tcPCO2 / EtCO2
56
Features from NIRS Sensing Device Pulsatile waveforms (e.g., AC/DC; Ecardiac.Etotal, etc.)
62
NIRS circulatory THb data (w/ confounding factor accounting)
64
NIRS circulatory THb data
66
NIRS tissue THb Calibration Parameter

*FIG. 7*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6456862 B
- US 7072701 B
- US 8078250 B
- US 8396526 B
- US 9988873 B
- US 8428674 B
- US 2020033258 A


### Non-patent literature cited in the description


- **DESEBBE OLIVIER et al.** Tissue Hemoglobin Monitoring Is Unable to Follow Variations of Arterial Hemoglobin During Transitions From Pulsatile to Constant Flow in Cardiac Surgery. *JOURNAL OF CARDIOTHORACIC AND VASCULAR ANESTHESIA*, 01 June 2014, vol. 28 (3), 668-673